# EUROPEAN PATENT SPECIFICATION

(11) **EP 1 624 796 B1**
(45) Date of publication and mention of the grant of the patent: **29.02.2012**
(21) Application number: 04733227.5
(22) Date of filing: 14.05.2004
(51) Int. Cl.: A61B 5/00

(54) **SYSTEM FOR THERAPY AND DIAGNOSIS COMPRISING IN COMBINATION NON-MECHANICAL AND MECHANICAL DISTRIBUTORS FOR DISTRIBUTION OF RADIATION**
SYSTEM FÜR BEHANDLUNG UND DIAGNOSE MIT KOMBINIERTEN NICHTMECHANISCHEN UND MECHANISCHEN VERTEILERN FÜR DIE VERTEILUNG VON STRAHLUNG
SYSTEME DE THERAPIE ET DE DIAGNOSTIC COMPRENANT, EN COMBINAISON, DES DISTRIBUTEURS NON-MECANIQUES ET MECANIQUES POUR LA DISTRIBUTION D'UN RAYONNEMENT

(30) Priority: 14.05.2003 SE 0301406; 14.05.2003 SE 0301410; 14.05.2003 SE 0301411; 16.05.2003 US 470856 P; 16.05.2003 US 470854 P; 16.05.2003 US 470855 P
(43) Date of publication of application: 15.02.2006
(73) Proprietor: SpectraCure AB, 226 43 Lund (SE)
(72) Inventor: JOHANSSON, Ann, S-224 72 Lund (SE); ANDERSSON ENGELS, Stefan, S-243 35 Höör (SE); JOHANSSON, Thomas, S-217 61 Malmö (SE); PALSSON, Sara, S-226 53 Lund (SE); SOTO THOMPSON, Marcelo, S-352 41 VÄXJÖ (SE); SVANBERG, Katarina, S-224 65 Lund (SE); SVANBERG, Sune, S-224 65 Lund (SE)
(74) Representative: KIPA
(86) International application number: PCT/SE2004/000758
(87) International publication number: WO 2004/100761

(56) References cited:
- EP-A1- 0 523 417
- EP-A1- 1 314 451
- EP-A2- 0 195 375
- EP-A2- 0 280 397
- WO-A1-02/074339
- US-B2- 6 416 531
- JOHANSSON T ET AL.: "Feasibility study of a system for combined light dosimetry and interstitiel photodynymic treatment of massive tumors", APPLIED OPTICS, vol. 41, no. 7, 1 March 2002 (2002-03-01), pages 1462-1468, United States

## Description

### Field of the Invention

The invention relates generally to a system for therapy and diagnosis in a subject. More particularly, the system and method relate to a system and method for tumour therapy and diagnosis in a human or animal. Even more particularly, the invention relates to a system and method for photodynamic therapy (PDT) and/or photothermal therapy (PTT) and/or photodynamic diagnosis (PDD) of a site on and/or in a body, wherein electromagnetic non-ionising radiation is conducted to the site for reaction with the radiation, wherein the system comprises at least one operation mode selector element for distribution of radiation from at least one source of radiation to a reaction site, and/or from the reaction site to at least one radiation sensor, respectively, and wherein the reaction site generally is a tumour site with a tumour, such as a malignant tumour.

### Background of the Invention

Within the field of medical therapy of tumour diseases, a plurality of treatment modalities has been developed for the treatment of malignant tumour diseases: operation, cytostatic treatment, treatment with ionising radiation (gamma or particle radiation), isotope therapy and brachytherapy employing radioactive needles are examples of common treatment modalities. In spite of great progress within therapy, the tumour diseases continue to account for much human suffering, and are responsible for a high percentage of deaths in western countries. A relatively new treatment modality, photodynamic therapy, commonly abbreviated PDT, provides an interesting complement or alternative in the treatment field. A tumour-seeking agent, normally referred to as a precursor or sensitizer, is administered to the body intravenously, orally or topically. It generally accumulates in malignant tumours to a higher extent than in the surrounding healthy tissue. The tumour area is then irradiated with non-thermal red light, normally from a laser, leading to excitation of the sensitizer to a more energetic state. Through energy transfer from the activated sensitizer to the oxygen molecules of the tissue, the oxygen is transferred from its normal triplet state to the excited singlet state. Singlet oxygen is known to be particularly toxic to tissue; cells are eradicated and the tissue goes in necrosis. Because of the localisation of the sensitizer to tumour cells a unique selectivity is obtained, where surrounding healthy tissue is spared. The clinical experiences, using in particular haematoporphyrin derivative (HPD) and delta aminolevulinic acid (ALA) have shown good results.

Sensitizers may also exhibit a further useful property; to yield a characteristic fluorescence signal when the substance is excited with visible or ultraviolet radiation. This signal clearly appears in contrast to the endogenous fluorescence of the tissue, which is also called autofluorescence, and is used to localise tumours and for quantifying the size of the uptake of the sensitizer in the tissue.

The limited penetration in the tissue of the activating radiation is a big drawback of PDT. The result is that only tumours less than about 5 mm in thickness can be treated by surface irradiation. In order to treat thicker and/or deep-lying tumours, interstitial PDT (IPDT) can be utilised. Here, light-conducting optical fibres are brought into the tumour using, e.g., a syringe needle, in the lumen of which a fibre has been placed.

In order to achieve an efficient treatment, several fibres have been used to ascertain that all tumour cells are subjected to a sufficient dose of radiation so that the toxic singlet state is obtained. It has been shown to be achievable to perform dose calculations of the absorptive and scattering properties of the tissue. E.g., in the Swedish patent SE 503 408 an IPDT system is described, where six fibres are used for treatment as well as for measurement of the light flux which reaches a given fibre in the penetration through the tissue from the other fibres. In this way an improved calculation of the correct light dose can be achieved for all parts of the tumour.

According to the disclosure of SE 503 408, the light from a single laser is divided into six different parts using a beamsplitter system comprising a large number of mechanical and optical components. The light is then focused into each of the six individual treatment fibres. One fibre is used as a transmitter while the other fibres are used as receivers of radiation penetrating the tissue. For light measurement light detectors are mechanically swung into the beam path which thus is blocked, and the weak light, which originates from the fibres that collected the light which is administered to the tissue, is measured.

However, such open beam paths result in a strongly lossy beamsplitting and the resulting losses of light drastically impair the light distribution as well as the light measurement. Furthermore, such a system must often be adjusted optically, which is also an important drawback in connection with clinical treatments. The system is also large and heavy and difficult to integrate into a user-friendly apparatus. Moreover, it is difficult to control the power of the light sent into each individual fibre, which makes the measurement results unreliable.

EP-A1-0523417 discloses a pipeline switch for distribution of radioactive emitters and/or test objects for radiotherapy, i.e. radioactive radiation treatment of a body. The emitters or test objects are conveyed within pipelines on flexible wires movable within the conduit. First pipes for conveying the flexible wires to the switch are on the one hand connected to a moveable switch element and second pipes further conveying the flexible wires to the body connected to a second, stationary switch element. The two switch elements are moveable relative each other and different constellations of pipelines are thus possible. However, when changing from one constellation to another, the flexible wire has to be retracted between each switching process, otherwise the relative movement of the switch element is obstructed. Switching times and treatment times are thus very long. Furthermore, the pipelines are not suitable for conducting radiation themselves, they just provide external protection and guidance to the flexible wires conveyed therein. The construction is also bulky and not suited for small optical fibres. Moreover, the arrangement of the disclosure is not suited for diagnosis, only for therapy, and no interactive co-operation is disclosed.

EP-A2-0280397 discloses a sterilizable endoscope having a central coherent fibre bundle for carrying an image to a viewing means. The fibre bundle is surrounded by light fibres. The proximate end of the endoscope is provided with a coupling means for aligning the optical fibre bundle with the optical system of the viewing means and for providing an interface with light transmitting means to transmit light from a light source along the light fibres to a body cavity to be inspected. The device can be used for detection of cancer cells and treatment thereof by phototherapy. A dye is attached to the tissue being examined and subsequently exposed to an exciting laser light frequency. Cancer cells will emit fluorescent light at a characteristic fluorescence frequency. The fluorescence light is detected and displayed on the video monitor and light with the same frequency as this fluorescent light is then transmitted through the light fibres to the cell for phototherapy treatment. However, only the use of a single wavelength light source is disclosed, it is thus not possible to have multiple diagnostics performed without manually exchanging the light source. Moreover, it is not possible to switch between different constellations of the light fibres, i.e. all fibres always have the same function (light in or light out). The coupling means mentioned in EP-A2-0280397 is only used to adjust the path of light through a two-part endoscope when it is assembled prior to use. In addition, different fibres are used for directing therapeutic light to a cancer location and to direct diagnostic light back through the endoscope. No distribution is performed between different operating modes. This solution offers for instance neither interactive treatment nor tomographic mapping of tumours.

WO-A1-02074339 discloses a device and method for photodynamic diagnosis of tumour tissue by using fluorescent cobalamins. These fluorescent cobalamins are used as diagnostic and prognostic markers (a) to distinguish cancer cells and tissues from healthy cells and tissues, and (b) to determine if an individual responds positively to chemotherapy using cobalamin-therapeutic bioconjugates. An apparatus is disclosed that includes a camera coupled to the proximal end of a surgical telescopic device. The surgical telescopic device is used for illuminating tissue with non-white light and detecting the emitted fluorescence for diagnostic purposes. The use of a dual light sources including a red (non-white)and a white light source is disclosed. The white light source is used for conventional illumination of the tissue. A switch is mentioned for switching between the alternative light sources. The switch might be voice-actuated, mechanically-operated (foot pedal), optically-operated, or electronically-operated. The switch is not described in more detail, except that a mirror or prism under mechanical or electromechanical control can be used to switch between the two light sources. Alternatively, a light source with two physically separated outputs is disclosed. In this case the light input to the surgical telescopic device has to be moved between the two outputs in order to switch illumination source for the tissue. The device is not suitable for therapy. Therapy has to be performed conventionally by a surgeon removing the cancerous tissue detected by means of fluorescence. Therefore, this device is not suited for interactive diagnosis and therapy. Furthermore, there is no indication for a switch suitable for switching between different modes of diagnosis or therapy. Furthermore, the disclosed device offers only substantially superficial diagnosis or treatment, interstitial tissue cannot be diagnosed or treated. The device is also limited to existing body cavities and has the drawback that endoscopic probes are bulky and large compared to single optical fibres.

EP-A2-0195375 discloses a catheter for laser angiosurgery. The device is used for detecting atherosclerotic plaque deposits by means of detecting fluorescent light as a reaction on excitation light sent through the catheter comprising optical fibres for this purpose. The same fibre may be used for sending excitation light to the plaque and for receiving fluorescent light from the plaque. When plaque is detected, it may be removed by sending high energy light through selected fibres in the catheter. However, this system is not suited for diagnosis or treatment of tumours. Fibres to be illuminated are selected by purely mechanical arrangements either moving the light source or the fibres in order to align the two towards each other. This device is also bulky compared to single fibres, similar to the above mentioned endoscope, bound to existing body cavities and works substantially superficial. Furthermore it is not selective, i.e. all tissue aimed at is destroyed, independently if it is noxious or healthy.

The disclosure of Johansson T et al
"Feasibility study of a system for combined light dosimetry and interstitial photodynamic treatment of massive tumors",
Applied Optics, United States, 2002
is considered as being the most relevant state of the art.

Thus, there is a need for a new compact device allowing distributing of radiation in a system for PDD, PDT and PTT for implementing a smart way of performing interactive interstitial treatment. One solution would be to use smart mechanical constructions for switching between different operating modes avoiding e.g. the lossy beamsplitters and allowing e.g. automatic calibration.

Such a mechanical solution to the above mentioned problems has been proposed in PCT/SE02/02050 published 22.5.2003, wherein a distributor for radiation having two discs rotating relative each other is described. The radiation distributor couples optical fibres between different operating modes by rotational movement of fibres in these discs relative each other. For switching between several light sources to one fibre going to the patient, an assembly with a total of four discs is described. There is a need to further reduce the size of the described solution in order to further minimise the size of the system.

However, although these purely mechanical constructions are improvements to the above described known IPDT system and although the above described problems are solved, these mechanical solutions have other limitations, related to e.g. mechanical inertia limiting the switching time between the different modes of a therapy and diagnosis system such as an interactive interstitial treatment system. There is also a desire to remove at least a certain number of mechanical switching elements in order to minimize costs for service. However, sometimes it is not economically defendable to replace all mechanical systems.

Thus, there is a need for a new system and/or compact device allowing distributing of radiation in a system for therapy and diagnosis in a human or animal, wherein the therapy and diagnosis comprises PDD, PDT and PTT, and wherein the system provides an optimal relationship between advantages such as reliable measurements, optimum of flexibility, cost-effectiveness, production efforts, but also that e.g. multiplexing, as described below, is possible

### Summary of the Invention

The present invention overcomes the above identified deficiencies in the art and solves at least the above identified problems by providing a system according to the appended patent claims, wherein a very practical and efficient implementation of interactive IPDT is achieved in that different radiation measurements for diagnostics and dosimetry can be performed in an integrated and simple way. An important application of the invention is interactive, interstitial photodynamic therapy, and/or interactive photothermal tumour therapy. According to the invention, the size of a system using existing radiation distributors, such as described in PCT/SE02/02050 is further reduced. Furthermore, the invention is improving the prior art by providing alternative solutions to the problems and drawbacks associated with the systems according to the prior art. Moreover, the system according to the invention combines the advantages of purely mechanical and purely non-mechanical solutions in a new and synergetic way. Non-mechanical elements have several advantages. Among others, these advantages comprise: high switching speed between different system operation modes (diagnosis, photodynamic therapy, thermal therapy); compactness and stability of the system; excellent radiation parameters; long life of the system due to no mechanical wear of the components and due to many more switching cycles during a life-cycle of the elements of the system, and low noise offering comfort to the user and patient, etc.. In combination with mechanical distributors for radiation, non-mechanical elements for distribution of radiation also provide increased flexibility.

The term "radiation" used hereinafter in this specification refers to radiation suitable for the field of the invention, i.e. for photodynamic therapy (PDT) and/or photothermal therapy (PTT) and/or photodynamic diagnosis (PDD). More specifically this radiation is "optical" radiation, i.e. non-ionising electromagnetic radiation within the wavelength-range of infrared (IR), visible or ultraviolet light. This also concerns radiation sources, radiation conductors, radiation sensors, radiation switches etc. within the scope of the embodiments and claims defining the invention, i.e. these sources, conductors or sensors for "radiation" are adapted to generate, conduct, measure, etc. the above-mentioned radiation.

According to one aspect of the invention, a system for therapy and/or diagnosis of a human or animal comprises at least one first radiation source for emission of a diagnostic radiation, at least one first radiation conductor adapted to conduct radiation to a site at or in said human or animal, at least one second radiation source for emission of a therapeutic radiation through at least one of said radiation conductors to said site, and at least one radiation detector, wherein at least one coupling element for coupling of radiation couples radiation from at least the first radiation source to the site and/or from said second radiation source to said site and/or from said site to said detector. The coupling elements are combinations of at least one translatory distributor comprising at least one translatory element being arranged in such a manner that radiation is coupled in different constellations by translatory movement of said translatory element between pre-determined positions, wherein radiation conductors are attached to said translatory element, and at least one rotary distributor comprising two rotary elements being arranged in such a manner that radiation is coupled in different constellations by rotational movement of said rotary element between pre-determined positions, wherein radiation conductors are attached to said elements, and at least one operation mode selector means for directing either said therapeutic radiation or said diagnostic radiation to said site through said at least one first radiation conductor.

As already mentioned, the system combines the advantages of non-mechanical elements with the advantages of mechanical elements in an optimal way. Among others, these advantages of radiation elements comprise: high switching speed between different system operation modes (diagnosis, photodynamic therapy, thermal therapy); compactness and stability of the system; excellent radiation parameters; long life of the system due to no mechanical wear of the components and due to many more switching cycles during a life-cycle of the elements of the system. Examples of the advantages of mechanical elements comprise: easy manufacturing; inexpensive; readily available technology; efficient radiation transmission; low crosstalk. Yet a further advantage is an adaptation to effective consideration of power management and compatibility of the system components. In general, the radiation used for therapy has a much higher effect/power than the diagnostic radiation, with at least a factor 10 in difference. The elements for switching/coupling this radiation have to be adapted to this effect. In general it is more cost-effective to use mechanical solutions for high power applications. Thus, the combination of elements suggested by the present application has a surprising synergetic effect that one does not realize immediately. Yet a further advantage is that according to one embodiment, the system is made more simple by using a therapeutic radiation source also for diagnostic radiation, thus saving a diagnostic radiation source. Finally switching times are provided that offer a convenient and comfortable treatment of a patient.

### Brief Description of the Drawings

In order to explain the invention in more detail, a number of embodiments of the invention will be described below with reference to the appended drawings, wherein
Fig. 1 is a schematic view illustrating an embodiment of the invention having a radiation distributor with two rotationally arranged discs and a radiation element for coupling between diagnostic radiation sources;
Fig. 2 is a schematic view illustrating another embodiment of the invention having two different translatory radiation distributors and one radiation element coupling three diagnostic radiation sources, wherein radiation guides are arranged interstitially inserted in a tumour;
Fig. 3 is a schematic view of a further embodiment of the invention comprising radiation combiners, an radiation switch and a translatory radiation distributor; and
Fig. 4 is a schematic view illustrating yet a further embodiment of the system according to the invention with a radiation distributor coupling diagnostic radiation sources by means of a translatory element.

### Description of embodiments

Different embodiments of the system according to the invention are now described with reference to the drawings. In order to simplify the description of the embodiments, reference numerals for similar elements shown in the drawings are not repeated throughout all the figures.

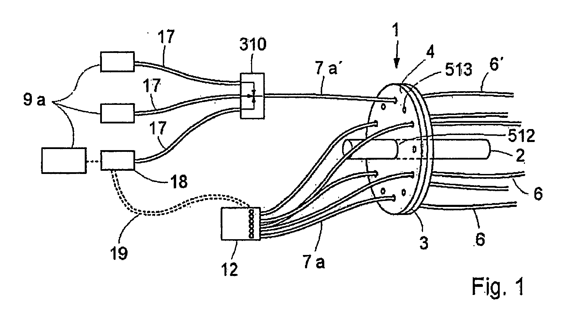

Fig. 1 is a schematic view illustrating an embodiment of the invention, wherein a rotary radiation distributor 1 comprises two flat and in proximity lying discs 3,4 made of, e.g., 1 cm thick material such as steel, aluminium/titanium/magnesium, a composite material etc. When composite material is used, this may reduce the thickness to some mm, depending on different parameters, such as the way of fastening the radiation conductors to the distributor. When a contact element, such as conventional optical fibre couplings are used for fixing the radiation conductors to the distributor, these couplings ensure the mechanical stability and define the size of the distributor elements. In case the radiation conductors are optical fibres directly attached to the distributor elements, the elements are more compact. This reasoning is valid for similar elements. In the case of a micromechanical realisation even smaller dimensions are obtained. The lighter the material is, the faster rotation of the discs between fixed positions is possible, while it is important that the discs at the same time are rigid and preferably durable. The same applies to the material of the other mechanical distributors described in this application. The two discs 3,4 are hereby arranged on an axis 2, wherein one of the discs is a fixed disc 4 and the other one is a turnable disc 3, wherein the terms "fixed" and "turnable" are merely for the purpose of simplifying and not for limiting the present description. The two discs 3, 4 are rotatable relative each other. In use the discs 3 and 4 are arranged in close proximity against each other, as shown in Fig.1.

Evenly distributed holes 513 lying on a circle are arranged in both discs (not shown in the Figs.) for fixation of radiation conductors 6, 6', 7a, 7a'. Preferably the diameter of the holes is 0.1 - 0.7 mm in case the radiation conductors are optical fibres directly attached to the disc. In order to attain a high precision, allowing the radiation conductors to be arranged exactly face to face, the holes of the two discs can be drilled together, e.g. with a centring tube. Alternatively, high precision cutter or drilling machines may be used for producing the discs or any other mechanical elements mentioned in this description. Then the common axis 2 is utilised arranged through centrally located holes 512 of the discs 3, 4. It is thus possible to achieve a very high precision when making the series of holes.

By employing discs drilled together, radiation conductors can be fixed in said discs, wherein an extra, thinner disc then can be turned slightly, preferably spring-loaded, so that all radiation conductors are simultaneously pinched in their positions without the need for any glue or other fixation means. Alternatively, the diameter of the holes is made larger than the diameter of the radiation conductors, wherein the holes can be dressed with an appropriate piece of tubing, or the ends of the radiation conductors can be supplied with a fitted hose. Alternatively, the ends of the radiation conductors can be flared or flanged into the holes or the holes may be equipped with appropriate SMA connector or other type of connectors for receiving radiation conductors. The same principle applies to the holes and fixation of radiation conductors in the translatory radiation distributors as described with reference to the following embodiments.

Preferably the radiation conductors are optical fibres, wherein different types of hoses or flexible tubes containing a radiation-conducting material are included. The radiation conductors should have such a length and be arranged in such a way that the discs can be turned a full turn (+-180 degrees) without problems. The direction of movement may be reversed to avoid the radiation conductors forming a spiral. The same principle applies to the translatory elements disclosed in this description, wherein the radiation conductors connected to the translatory elements should have such a length that the function of the translatory elements or the radiation conductors is not negatively influenced. Moreover, the length of the radiation conductors should be sufficiently long, that the positioning of the distal ends of the patient radiation conductors are not negatively influenced.

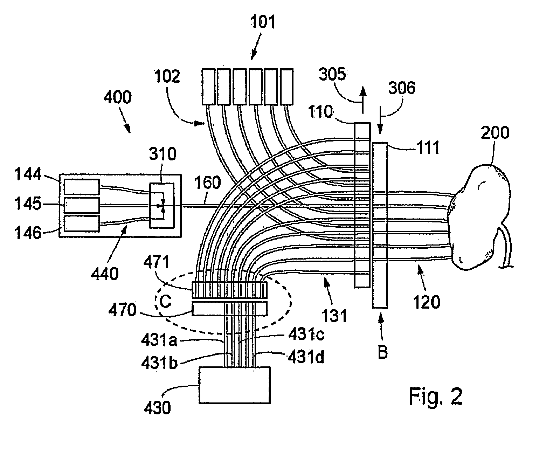

According to this embodiment of the invention, a plurality of first radiation conductors 6 in a system for PDD, PDT and PTT are arranged in turnable disc 3 for conduction of radiation to and from a reaction site 200 (shown e.g. in Fig. 2). By a reaction site we in the present context mean a site where photodynamically active compounds will react in a tumour when subject to therapy e.g., by being forwarded through the lumen of injection needles which are placed in the tumour, these radiation conductors 6 are then fixed in the reaction site 200. Then the radiation conductors are moved forward to arrive outside the distal end of the needle. The same radiation conductor 6 is used continuously during the treatment for integrated diagnostics and dosimetry as well as to avoid that the patient be subjected to multiple pricks.

The holes 513 in the fixed disc as well as in the turnable disc are arranged on a circular line, wherein the circle radius on one disc equals the circle radius on the other disc. The holes on one disc are equally distributed along the circle line with an angular separation of v1 = (360/n1) degrees, where n1 equals the number of holes, and the holes of the other disc are equally distributed along the circle line with an angular separation v2 equalling (360/n2) degrees. The first ends of the first radiation conductors 6 are fixed in the holes of the turnable disc 3, and first ends of the second radiation conductors 7a are fixed in the holes of the fixed disc 4. In order to make the holes, and thereby the radiation conductors in both discs connectable to each other in different constellations by turning of the turnable disc 3, n2 is selected to be a multiple of n, in such a way that n2 is obtained as an integer larger or equal to 1. Suitably the number of holes in the fixed disc is chosen from two to more than six, e.g. two, three, four, five, six, seven, eight, nine or ten.

According to the currently described embodiment, six holes are arranged in the turnable disc 3 and twelve holes are arranged in the fixed disc 4.With six first radiation conductors 6 the angular separation of the holes will accordingly become 60 degrees in the turnable disc 3 and with twelve holes arranged in the fixed disc 4 the angular separation will become 30 degrees for the second radiation conductors 7a.

The coupling of diagnostic radiation from diagnostic radiation sources 9a to a single radiation conductor 7a' is accomplished by means of a radiation combiner 310. For switching to a diagnostic operation mode, the therapeutic radiation sources (not shown in Fig. 1) are switched off and subsequently one of the three diagnostic radiation sources 9a is activated. Thus, diagnostic radiation is conducted to combiner 310 via radiation conductors 17, where the radiation from the active diagnostic radiation source is coupled to the output of the combiner leading via the radiation conductor 7a' to disc 4, where it is coupled to one radiation conductors 6' going to a tumour site 200 in a patient.

In order to facilitate the comprehension of the invention the following description of a preferred embodiment of the distributor of the system according to Fig. 1 comprises a rotary radiation distributor, which relates to six first radiation conductors 6 arranged in the turnable disc 3 for conduction of radiation to and from the reaction site 200.

Thus, the turnable or rotatable disc 3, as well as the fixed disc 4, have six holes 513 for corresponding second radiation conductors, and, for disc 4, in addition, six further holes for second radiation conductors being connected to therapeutic radiation sources (not shown in Fig. 1). All these radiation conductors can release radiation to the reaction site 200 and receive radiation from said site. Thus, several measurements can be recorded and read out simultaneously.

By turning the turnable disc 3 the first and the second radiation conductors become connectable to each other in different constellations. An exact positioning of the opposing radiation conductors in the distributor 1 is facilitated by arranging means for stopping the turnable disc 3 in pre-determined angular positions, for instance, grooves may be arranged in the axis 2 for catching a spring-loaded ball arranged in the turnable disc 3 (not shown in the Figs.) or an angular detector on the rotatable disc can be used. Alternatively electronic regulation using stepper motors or servomotors may be used for this purpose, also in combination with the "seventh hole" method described below.

In order to allow a fast and efficient switching between a diagnostic operation mode and a therapeutic operation mode, every second of the second radiation conductors of the distributor 1 according to the invention, are divided into a first and into a second series. Both series of holes are arranged on the same circle, but displaced by 30 degrees with regard to each other. A specific radiation conductor in the first series of every other second radiation conductor is arranged for emitting radiation from at least one radiation source. The other, non specific radiation conductors in the first series of second radiation conductors are arranged for conduction of radiation to at least one radiation sensor 12. The second series of every other second radiation conductor is for therapeutical purposes arranged to emit radiation to the reaction site 200 from at least one radiation source.

The radiation conductors are preferably optical fibres, which in the distributor 1 shown in Fig 1 are connected to the fixed disc 3 as well as the turnable disc 4. Out of the fibres, which are connected to the turnable disc 4, six fibres can be used for diagnostic purposes and six can be used of therapeutical purposes. However, in the diagnostic operation mode, radiation from one to more than three modalities 9a can be employed.

With reference to Figs. 1 only radiation conductors which are coupled for diagnosis to a fixed disc are for clarifying purposes shown; the other radiation conductors are not shown although they are coupled to said disc.

By turning the turnable disc 3 by 30 degrees the fibres 6 which are optically coupled to the tissue of the patient can be employed for therapy as well as diagnostics and measurements. One radiation conductor 7a' out of every second radiation conductor fixed on disc 4 is in the diagnostic operation mode connected to different radiation sources for diagnostics, while the other five radiation conductors 7a receive signals, which are related to the interaction of these radiation sources with the tissue. Radiation conductors (not shown in Fig. 1, whereas the holes 513 are shown) are connected to therapeutic radiation sources, e.g. lasers, whereas radiation conductors 7a are connected to radiation detectors. Radiation conductors 17 are coupled to diagnostic radiation sources 9a.

Since intensity as well as spectral resolution is of interest, the distal ends of these five radiation conductors 7a are arranged in a slit-like arrangement so that they overlap the entrance slit and/or constitute the entrance slit of the radiation sensor 12, which may be a compact spectrometer or other type of detector and is supplied with a two-dimensional detector array or one to several one dimensional detector arrays. The recording range of the spectrometer is preferably within the range of approximately 400 to 900 nm. Each of the radiation conductors 7a can of course be connected to an individual radiation detector 12 in the form of a spectrometer or another type of detector, e.g. a compact integrated spectrometer.

The assembly 1 is shown with the two discs 3, 4 on a common axle 2 and the combiner 310 for coupling different diagnostic radiation sources. In this way a compact and robust construction is obtained for switching between the diagnostic radiation sources.

Preferably one of the radiation sources 9a is a laser of the same wavelength as the ones utilised for the laser irradiation for photodynamic tumour therapy, but could be of lower output power.

Certain of the radiation sources 9a are utilised in order to study how radiation (light) of the corresponding wavelength is penetrating through the tissue of the tumour. When radiation from a radiation source is transmitted through the particular radiation conductor via combiner 310 and the discs 4, 3 into the tissue, one radiation conductor 6' of the first radiation conductors 6, which is the one opposing the radiation conductor 7a', will function as a transmitter in the tumour, and the other five radiation conductors 6 in the tumour will act as receivers and collect the diffuse flux of radiation reaching them. The radiation collected is again conducted via the discs 3, 4 and via radiation conductors 7a to the radiation sensor 12 and five different radiation intensities can be recorded on the detector/detectors/ detector array.

When the turnable disc 3 is turned by 60 degrees, the next radiation conductor 6 to the patient will get the role as transmitter, and the five others become the receivers for a new radiation distribution. After four further turns of the turnable disc 3, each by 60 degrees to the following radiation conductor 6 in the patient, radiation flux data for all remaining combinations of transmitters/receivers have been recorded. Thus, in total 6 x 5 = 30 measurement values are obtained and can be used as input data for a tomographic modelling of the radiation dose build up in the different parts of the tumour during the course of the treatment. Furthermore, by switching through the three radiation sources 9a, by means of switching the appropriate radiation source on or off, these 30 measurement values are multiplied by the number of radiation sources, resulting in 90 tomographic measurement values.

In addition to a specific wavelength, radiation from a white light source and/or broadband light emitting diodes and/or line light sources can be coupled into the particular active radiation conductor in radiation distributor 1. On passage through the tissue to the receiving radiation conductor 6 in the patient, the well-defined spectral distribution of the radiation source will be modified by the tissue absorption. Then, oxygenated blood yields a different signature than non oxygenated blood, allowing a tomographic determination of the oxygen distribution utilising the thirty different spectral distributions which are read out, five spectra at a time in the six possible different constellations on rotation of the turnable disc 3 during a diagnostic investigation. Such a determination of the oxygenation in the tumour is important, since the PDT process requires access to oxygen in the tissue.

Finally, a light source for red, blue/violet or ultraviolet light, e.g. a laser, can be coupled to the particular active radiation conductor in radiation distributor 1. Then fluorescence is induced in the tissue, and a sensitizer administered to the tissue displays a characteristic red fluorescence distribution in the red/near-infrared spectral region. The strength of the corresponding signal allows an approximate quantification of the sensitizer level in the tissue.

Since the short wavelength light (ultraviolet or blue/violet light) has a very low penetration into the tissue, the induced fluorescence will only be measured locally at the tip of the radiation conductor. For this task there is in this case for the corresponding radiation source 9a at the distal end of the particular radiation conductor 17 a beamsplitter 18, connected via the radiation conductor 17 and which is preferably a dichroic beamsplitter, transmitting the exciting radiation but reflecting the red-shifted fluorescence radiation. This reflected radiation is focused into the distal end of a conveying radiation conductor 19, the other end of which is connected to the radiation sensor 12, which records the fluorescence radiation distribution. A suitable self-contained fluorosensor is described in Rev. Sci. Instr. 71, 3004 (2000). Such a system with dichroic beamsplitters may also in a similar way be implemented by means of the other distributor system as shown in Figs. 2 to 4.

By rotating the turnable disc 3, the fluorescence that is a specific function of the concentration of the sensitizer, can be measured sequentially at the tips of the six radiation conductors 6. Since the sensitizer is bleached by the strong red treatment radiation, being particularly strong just around the tip of the radiation conductor 6 conducting radiation to the patient, it is essential to make this measurement before the start of the treatment.

If the tips of the radiation conductors 6 in addition are treated with a material, the fluorescence properties of which are temperature dependent, sharp fluorescence lines are obtained upon excitation, and the intensity of the lines and their relative strength depend on the temperature of the tip of the radiation conductor 6, being employed for treatment. Examples of such materials are salts of the transition metals or the rare earth metals. Thus also the temperature can be measured at the six positions of the six radiation conductors, one at a time. The measured temperatures can be utilised to find out if blood coagulation with an associated radiation attenuation has occurred at the tip of the radiation conductor 6, and for studies regarding the utilisation of possible synergy effects between PDT and thermal interaction. Since the lines obtained are sharp, they can be lifted off the more broad-banded fluorescence distribution from the tissue.

The concentration of the sensitizer can for certain substances be measured in an alternative way. Then the red radiation used for the radiation propagation studies is used to induce near-infrared fluorescence. This fluorescence penetrates through the tissue to the tips of the receiving radiation conductors 6, 120, 142, and are displayed simultaneously as spectra obtained in the radiation sensor 12. A tomographic calculation of the concentration distribution can be performed based on in total thirty measurement values.

After diagnostic measurements and calculations have been performed, the fibres 6 optically coupled to the tissue of the patients can be utilised for therapy by rotation of the turnable disc 3 by 30 degrees. Therapeutic radiation sources are thus coupled to the patient fibres 6. The therapeutic radiation sources are preferably Laser sources with a wavelength, which is adapted to the absorption band of the sensitizer. At the photodynamic tumour treatment a dye laser or a diode laser is preferably used, with a wavelength which is selected with regard to the sensitizer employed. For Photofrin^{®} the wavelength is 630 nm, for delta aminolevulinic acid (ALA) it is 635 nm and for phthalocyanines it is around 670 nm. The individual lasers are regulated during the treatment to a desirable individual output power. If desired, they may have built-in or external monitoring detectors.

The therapeutical treatment can be interrupted and new diagnostic data can be processed in an interactive method until an optimal treatment has been reached. This method can include synergy between PDT and hyperthermia, where an increased temperature is reached at increased fluxes of laser radiation. The whole process is controlled using a computer, which does not only perform all the calculations but also is utilised for regulation.

This present embodiment has the advantages of being cheap, all diagnostic radiation sources may be switched on at the same time or multiplexed, there is little risk of "blooming" (detector in permanent saturation) of the radiation detector 12, there is no moving element or mechanical part for switching between the detector radiation sources, optical fibres with large diameters may be used, and the system has low radiation losses, i.e., it has a sound "photoneconomics". Fig. 2 is a schematic view illustrating another embodiment 400 of the invention having two different translatory radiation distributors B, C and one radiation element 310 coupling three diagnostic radiation sources, wherein radiation guides 120 are arranged interstitially inserted in a tumour 200.

The coupling of diagnostic radiation from diagnostic radiation sources 144-146 to a single radiation conductor 160 is accomplished by means of an radiation combiner 310. For switching to a diagnostic operation mode, the therapeutic radiation sources 101 are switched off and subsequently one of the three diagnostic radiation sources 144-146 is activated. Thus, diagnostic radiation is conducted to combiner 310, where the radiation from the active diagnostic radiation source is coupled to the output of the combiner leading via the radiation conductor 160 to a transversal radiation distributor 110, 111, where it is coupled to one of radiation conductors 120 going to a tumour site 200 in a patient.

A first translatory distributor B for radiation comprises two, in close proximity to each other lying, longitudinal translatory elements 110, 111 made of, e.g., 1 cm thick steel, however lower thicknesses are possible, as mentioned above. The longitudinal translatory elements are hereby arranged in such a manner that they may move translatory relative to each other in such a manner that a plurality of radiation conductors 160 or 102, 131 respectively, such as optical fibres, being attached to holes in the first translatory element 110 are coupled to a second plurality of fibres 120, 431a-431e respectively, being attached to holes in the second translatory element 111, 470 by appropriately positioning the two elements relative to each other. The system 400 shown in Fig. 2 comprises two such radiation distributors B and C comprising the translatory elements 110, 111, 470, 471. These elements are shown as longitudinal elements in Fig. 2. However, they may have another geometrical configuration. Furthermore, at least one of the elements may be integrated into a housing etc. The elements may be sledges, for coupling either treatment radiation or diagnostic radiation to a patient.

In the diagnostic position radiation is coupled to at least one radiation detector 430. The diagnostic part of system 400 comprises three diagnostic radiation sources 144-146, which are coupled to a single output radiation conductor 160 by means of an radiation combiner, in such a manner that an active diagnostic radiation source is coupled to radiation conductor 160 and further to the site in the patient to be treated via translatory radiation distributor B. This diagnostic operation mode will be described in more detail below. Furthermore a plurality of diagnostic radiation sources may be used simultaneously. In this case several diagnostic radiation sources may be modulated, so that the diagnostic radiation may be detected simultaneously by means of e.g. a lock-in method or by multiplexing the signals, wherein the therapeutic radiation preferably, but not necessarily, is shut off in diagnostic operation mode.

Main radiation distributor B comprises two translatory elements 110, 111. The two translatory elements 110, 111 are displaceable with relation to the other translatory element, as indicated by the arrows 305, 306. The displacement is controlled in such a manner that a plurality of radiation conductors 120 lead radiation to and from a tumour site 200 in a patient. Main radiation distributor B switches between the diagnostic operation modes and the therapeutic operation mode. The radiation conductors 120 leading to and from the patient are fixed to the translatory element 111. The translatory element 110 of the main radiation distributor B comprises a (3N-1) to N radiation distributor, wherein N is the number of radiation conductors 120 to/from the patient fixed in translatory element 111 and (3N-1) is the number of radiation conductors fixed in translatory element 110 of which N are radiation distributors 102 coupled to radiation sources 101 and 2(N-1) are radiation distributors 131 coupled to radiation detector 430, and one, 160, is coupled to the diagnostic radiation sources 144-146 via combiner 310.

In the therapeutic operation mode, B is adjusted translatory in such a manner that treatment radiation originating from the radiation sources 101 is coupled to radiation conductors 102. These radiation conductors, preferably light guides or optical fibres, are coupled to translatory displacement element 110. Element 110 is aligned with translatory displacement element 111 in such a manner that the radiation from radiation sources 101 is coupled to radiation conductors 120 and further to the treatment site 200 in the patient.

In diagnostic operation mode the at least one active diagnostic radiation source 144-146 is coupled to fibre 160 by means of combiner 310. Main radiation distributor B is in diagnostic operation mode adjusted such that one of the N patient fibres 120 is coupled to a diagnostic radiation conducting radiation conductor 160, as illustrated in Fig. 2. This is accomplished by transversally sliding the translator elements 110, 111 relative each other, as indicated by arrows 305, 306. The radiation, which is being transmitted back from the site in the patient through the remaining (N-1) fibres from the plurality of fibres 120, is also called diagnostic radiation. This diagnostic radiation is coupled to (N-1) radiation conductors from a plurality of radiation conductors 131 leading to the radiation detector 430. Subsequently, the radiation distributor B is adjusted in such a way that another of the N patient fibres 120 is coupled to diagnostic radiation emitting fibre 160. This is accomplished by once again sliding the translator elements 110, 111 transversally relative each other, as indicated by arrows 305, 306. In this way another set of (N-1) fibres is coupled to (N-1) radiation conductors from a plurality of radiation conductors 131 leading to the radiation detector 430. This is repeated N times, until all N coupling combinations of fibre 160 to the N patient fibres, is accomplished. In case a plurality of n diagnostic radiation sources is present in the system, the N measurements are carried out with each of the n radiation sources, which results in (N*n) diagnostic measurements, each measurement delivering (N-1) measurement values. Alternatively to the sequence described above, the n radiation sources are applied subsequently, before switching to the next input fibre to the patient. The detector may be a single detector or a plurality of detectors or an array detector.

A further translatory radiation distributor C having elements 470, 471 is used for minimising the number of radiation conductors leading to detector 430. Distributor C comprises two translatory elements 470, 471. The two translatory elements 470, 471 are displaceable with relation to the other translatory element respectively. A plurality of (N-1) radiation conductors 431a-431e, corresponding to the (N-1) radiation conductors conducting diagnostic radiation from the patient, are fixed to the translatory element 470 and lead to the detector 430. 2*(N-1) radiation conductors 131 lead from the translatory element 110 to the translatory element 471. Radiation distributor C is adjusted in such a manner that only the active (N-1) radiation conductors of the plurality of conductors 131 are coupled to the detector 430 through radiation conductors 431. Alternatively, the translatory element 471 may be integrated with the translatory element 110 and the translatory element 470 may be integrated with the translatory element 111 (not shown in the Figs.). In this way, the one and same translator may be used for therapy and diagnostic measurements.

N = 6 and n = 3 in the exemplary embodiment given above. However, other numbers of N and n are equally possible.

For calibration purposes of at least the mechanical part of a system according to embodiments of the present invention comprising at least one mechanical radiation distributor, a 7th hole may be present in translator 111 or similar elements. Preferably this hole is located exactly between two fibres 120 on translator 111, with reference to the linear translator shown in Fig. 2. Concerning the disc 4 shown in Fig. 1, the 7^{th} hole is preferably located anywhere in between holes on the disc 4 to which the radiation conductors 7a are attached. The 7^{th} hole is used to exactly define the position of an input fibre in a hole on the opposite element of a radiation distributor. The 7^{th} hole is either directly equipped with a radiation sensor or connected to a radiation sensor for detecting radiation transmitted from a radiation conductor facing the 7^{th} hole from the opposite side. In this way the positioning of the elements of a radiation distributor may be calibrated. For instance the position of the 7^{th} hole may be used to zero the position of stepping motors driving these elements. The additional hole may equally be used to calibrate the position of translatory element C or any other translatory or rotary element of the embodiments of the system according to the invention in the same way.

This embodiment has the advantages of avoiding torsion of the radiation conductors, there occurs no "blooming" of the detectors, and it allows a compact and flat implementation including a very compact layout of a device for performing the method, which method can be carried out with the system according to the invention. Furthermore, there is no moving element or mechanical part for switching between the detector radiation sources, optical fibres with large diameters may be used, and the system has low radiation losses, i.e., it has a sound "photon economy".

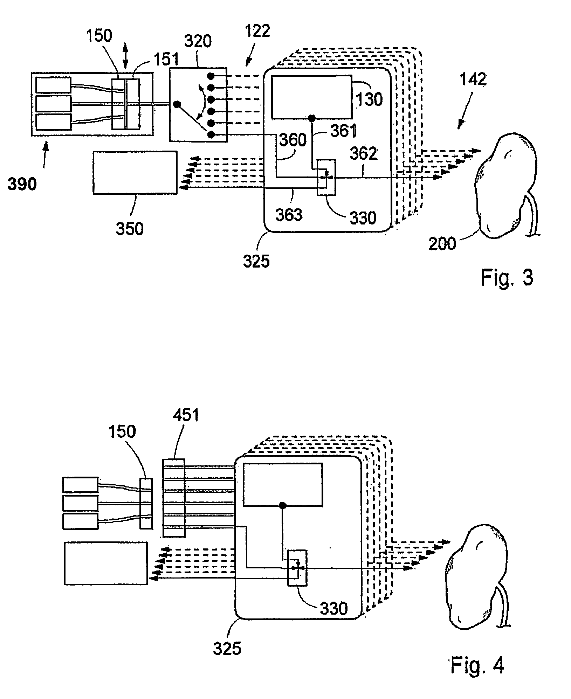

Fig. 3 is a schematic view of a further embodiment of the invention. The system shown in Fig. 3 comprises radiation combiners, a radiation switch and a translatory radiation distributor. More particularly, a system is shown comprising a translatory 3x1 element 150, 151 and a radiation 1x6 switch 320 as well as a radiation combiner 330 as an operation mode selector in six modules 325. For interstitial treatment six therapeutic radiation sources 130, preferably laser radiation modules, are coupled to the six radiation combiners 330. Each radiation combiner 330 works in such a manner that the therapeutic radiation in therapy operation mode is coupled through the corresponding radiation conductor 142 to the treatment site 200. For switching to the diagnostic operation mode, the therapeutic radiation source is switched off and subsequently one of the three diagnostic radiation sources in box 390 is activated. Thus, diagnostic radiation is conducted to the translatory 3x1 element 150, where the radiation from the active diagnostic radiation source is coupled to the output of the translatory 3x1 element 151, leading to the radiation switch 320. The radiation switch 320 couples the input radiation to an output radiation conductor 122 leading to the corresponding radiation combiner 330 comprised in one of modules 325. From combiner 330, the diagnostic radiation is sent to the treatment site via a radiation conductor 142 connected to combiner 330, as shown in Fig. 3. Thus the diagnostic radiation is spread in the treatment site and partly to the remaining five radiation conductors 142 for diagnostic measurements and partly reflected back. The diagnostic radiation from the patient is via combiner 330 sent to radiation detector 350. Thus five (=(N-1)) measurement values are obtained. Subsequently the radiation switch 320 switches the incoming diagnostic radiation from the radiation source indicated at 310 to the next combiner 330 comprised in the next module 325. Thus five further measurement values are obtained. This measurement procedure is repeated until all six modules 325 have been activated, resulting in six times five =30) measurement values. These thirty measurement values obtained may be used as input data for a tomographic modelling of the radiation dose build up in the different parts of the tumour during the course of the treatment. This measurement procedure may be repeated with the remaining diagnostic radiation sources, yielding three times thirty (n*N*(N-1))or ninety tomographic measurement values. Also the diagnostic radiation reflected at site 200 from the illuminating radiation connector may be used for diagnostic purposes. This embodiment allows fast treatment cycles due to fast switching times. Furthermore the diagnostic radiation does not need to be switched off during e.g. therapy, as e.g. the radiation switch may disconnect the radiation beam path, or the translatory elements 150,151 may be adjusted so that no coupling from an diagnostic input radiation conductor to corresponding output radiation conductor occurs. During the diagnostic operation mode collecting the ninety tomographic measurement values, all the diagnostic radiation sources may also be switched on, as there is no coupling between the different diagnostic radiation sources indicated at 310. Furthermore, this embodiment avoids torsion of the radiation conductors going to the site in the patient as well as other mechanical movement that may be induced by mechanical movement of the radiation conductors going to the site in the patient.

The combiner 330 may be a fibre combiner commercially available from, e.g., Polymicro Technologies.

As a basis for the radiation switch 320 one may use a commercially available radiation fibre switch from Piezosystem Jena Inc. The working principle of the combiner 330 is described below in more detail. The combiner 330 may also be based upon a commercially available fibre combiner from Polymicro Technologies. The combiner is connected to two source radiation conductors 360 and 361 one detection radiation conductor 363, and one patient radiation conductor 362, wherein radiation is mainly transmitted along these radiation conductors in the directions as indicated by arrows on the corresponding radiation conductors shown in Fig. 3 and 4. The source radiation conductors 360 and 361, and the detection radiation conductor 363 are fused together to the patient radiation conductor 362 along a certain length being shorter than the total radiation length of the combiner 330. Thus radiation is transmitted via the source radiation conductors to the patient radiation conductor, while radiation from the patient radiation conductor is transmitted in the opposite direction to the detection radiation conductor. In the embodiment according to Fig. 3, one source radiation conductor is connected to the therapeutic radiation source 130, and the second source fibre is connected to the diagnostic radiation source 110 and the detection radiation conductor is connected to the radiation detector 350. The combiner 330 can be made to transmit the main part of the diagnostic radiation emerging from the tissue site 200 via the patient radiation conductor 362 to the detection fibre 363, assuring an efficient use of the occasionally faint diagnostic radiation. The combiner 330 does not transmit radiation directly from the source fibres 360, 361 connected to the diagnostic and therapeutic radiation sources respectively to the detection fibre 363 and thus to the radiation detector 350.

Fig. 4 is a schematic view illustrating yet a further embodiment of the system according to the invention with a radiation distributor coupling diagnostic radiation sources by means of a translatory 3x6 element. Similar to the system shown in Fig. 3, the system shown in Fig. 4 comprises radiation combiners and a translatory radiation distributor, but the radiation switch is skipped. More particularly, a system is shown comprising a translatory 3x6 element 150,451 as well as a radiation combiner 330 as an operation mode selector in six modules 325. For interstitial treatment six therapeutic radiation sources 130, preferably laser radiation modules, are coupled to the six radiation combiners 330. Each radiation combiner 330 works in such a manner that the therapeutic radiation in therapy operation mode is coupled through the corresponding radiation conductor 142 to the treatment site 200. For switching to the diagnostic operation mode, the therapeutic radiation source is switched off and subsequently one of the three diagnostic radiation sources 110 is activated. Thus, diagnostic radiation is conducted to the translatory 3x6 element 150, where the radiation from the active diagnostic radiation source is coupled to the output section 451 of the translatory 3x6 element. Element 451 is directly connected to radiation conductors 122 leading to the corresponding radiation combiner 330 comprised in one of modules 325. From combiner 330, the diagnostic radiation is sent to the treatment site via a radiation conductor 142 connected to combiner 330, as shown in Fig. 3. Thus the diagnostic radiation is spread in the treatment site and partly to the remaining radiation conductors 142 for diagnostic measurement and partly reflected back. The diagnostic radiation from the patient is via combiner 330 sent to radiation detector 350. Thus five (=(N-1)) measurement values are obtained. Subsequently another diagnostic radiation source may be activated from the radiation source 310 to the next combiner 330 comprised in the next module 325. Thus five further measurement values are obtained. This measurement procedure is repeated until all six modules 325 have been activated, resulting in six times five (=30) measurement values. These thirty measurement values obtained may be used as input data for a tomographic modelling of the radiation dose build up in the different parts of the tumour during the course of the treatment. This measurement procedure may be repeated with the remaining diagnostic radiation sources, yielding three times thirty (N*(n-1)) or ninety tomographic measurement values. Also the diagnostic radiation reflected at site 200 from the illuminating radiation connector may be used for diagnostic purposes.

Furthermore the diagnostic radiation sources 310 may be modulated, so that the diagnostic radiation may be detected simultaneously by means of, e.g., a lock-in technique or by multiplexing the signals, wherein the therapeutic radiation is preferably shut off in diagnostic operation mode.

Diagnostic measurements may be made simultaneously according to the present embodiment by multiplexing the diagnostic radiation sources. As shown in Fig. 4, three diagnostic radiation sources are coupled simultaneously to radiation conductors 122, except in the end positions of the translatory elements relative each other. Element 150 has to be moved nine times in the present embodiment for obtaining the mentioned ninety measurement values.

Alternatively, the radiation conductors in part 150 are attached to part 150 with a different interval space between the radiation conductors than the interval space of radiation conductors 122 in element 451. In this case, element 150 has to be moved eighteen times. However, this has the advantage that the diagnostic radiation sources do not need to be switched off between the measurements, as two of the three radiation conductors in element 150 are alternatingly disconnected from radiation conductors 122 (by translatory displacement) and only one radiation conductor 122 conducts radiation to the corresponding module 325.

This embodiment allows a very compact construction, it is very cheap to implement and there is only one moving part and no torsion in the radiation guides.

For calibration purposes of the system according to the invention, the overall performance of the system is recorded prior to the treatment by direct measurements on a calibrated tissue phantom made of, e.g., a sterile intralipid-water solution and/or a sterile solid phantom made of, e.g., Delrin^{®}. The performance of the therapeutic radiation sources may either be monitored by internal and/or external power meters.

The radiation switches described may work according to different principles. One is the switching by direct radiation conductor movement actuated by piezoelectric movement of the radiation conductor in relation to output radiation conductor. Another is the switching by microradiation beam deflection, which may be based on micromechanical components, such as microprisms or mirrors or by acousto-optical means, deflecting an optical beam to different output/input fibres. The switching and beam deflection is based on optical principles without mechanical movement of components such as prisms or mirrors. Examples of non-mechanical switching principles are for instance beam deflection by an acousto-optical means based on sound generated Bragg deflection, or acousto magnetic means, or by an electrically controlled variation of the refractive index of a material through which the beam travels, thereby deflecting an optical beam to different output/input fibres. Examples for materials having a variable refractive index suitable for electro-optical switches are e.g. LiNbO_{3,} , LiTaO₃ , GaAs, HgS, CdS, KDP, ADP or SiO₂ . The Agiltron™ company provides commercially available optical switches of this type, namely the CrystaLatch™ Solid-State Fiber Optic Switch family or the NanoSpeed™ Optical Switch Series.

The therapeutic radiation sources are preferably laser sources with a wavelength, which is adapted to the absorption band of the sensitizer. At the photodynamic tumour treatment a dye laser or a diode laser is preferably used, with a wavelength which is selected with regard to the sensitizer employed. For Photofrin^{®} the wavelength is 630 nm, for delta-aminolevulinic acid (ALA) it is 635 nm and for phthalocyanines it is around 670 nm. The individual lasers are regulated during the treatment to a desirable individual output power. If desired, they may have built-in or external monitoring detectors.

The therapeutical treatment can be interrupted and new diagnostic data can be processed in an interactive method until an optimal treatment has been reached. This method can include synergy between PDT and hyperthermia, where an increased temperature is reached at increased fluxes of laser radiation. The whole process is controlled using a computer, which does not only perform all the calculations but also is utilised for regulation.

The radiation distributors described are preferably driven by stepper motors / servomotors in order to move between the different constellations.

Naturally, diagnosis and therapy may also be performed at the same time, if so desired. With an appropriate number of radiation conductors going to the tumour, for instance the above mentioned six radiation conductors for therapeutic irradiation plus four radiation conductors for simultaneously diagnosing the effect of the therapeutic light, it is possible to directly regulate therapy in real-time. This is of particular interest when performing therapy on sensitive organs that are not to be damaged by the therapeutic optical radiation. Of course it is a goal to only destroy tumour tissue. In the given example, the six radiation conductors illuminate the tumour tissue into which the distal ends of the six radiation conductors are placed. The four diagnostic radiation conductors are also placed into the tumour tissue at appropriate locations and pick up both the excitation radiation from the therapeutic radiation conductors scattered in the tumour tissue and the fluorescent radiation resulting in the tumour tissue. This picked-up radiation may be analysed in a spectrometer and be used for regulating the therapeutic radiation source. These, for example, four extra radiation conductors can be placed in-between the six radiation conductors for therapeutic irradiation. When the six radiation conductors are connected to the radiation sources by means of the above-described arrangements, the four extra radiation conductors are suitably automatically connected to the radiation detector, e.g. because of the arrangement of the translatory slides or rotating discs.

The present invention has been described above with reference to specific embodiments. However, other embodiments than the preferred above are equally possible within the scope of the appended claims, e.g. different shapes of the translatory elements, different radiation coupler elements than those described above or other elements described in this description than those described above, performing the above method by hardware or software, fluorescence or temperature measurements described in one embodiment, etc. Moreover, the translatory elements may be further minimised by using micromechanical technologies for constructing the elements. Thus, one realisation of the elements may be provided by a Micro-Electro-Mechanical System (MEMS) produced by microfabrication technology. The elements described may work according to different principles. One is the switching by direct fibre movement actuated by piezoelectric movement of the fibre in relation to output fibres. Another is the switching by microoptical beam deflection, which may be based on micromechanical components, such as microprisms or mirrors deflecting an optical beam to different output/input fibres. Piezosystem Jena Inc or Pyramid Optics Inc. provide suitable components based on the latter micromechanical principles.

Furthermore, the term "comprises/comprising" when used in this specification does not exclude other elements or steps, the terms "a" and "an" do not exclude a plurality and a single processor or other units may fulfil the functions of several of the units or circuits recited in the claims.

## Claims

1. A system for interactive interstitial photodynamic or photothermal tumour therapy or tumour diagnosis of a human, comprising
at least a diagnostic (9a, 144-146) and a therapeutic (101) light source for emission of light within the wavelength-range of infrared (IR), visible or ultraviolet light;
at least one light detector (12, 430), for detection of light; and
a plurality of first optical fibres (6) adapted to conduct light to or from a tumour site (200) of said human, whereby one therapeutic light source is coupled to each of said optical fibres for transmission of said therapeutic light to said tumour site through each of said optical fibres, during which the diagnostic light sources (9a) are inactivated, whereby distal ends of the optical fibres are positioned at different interstitial locations of the tumour site in order to enable an effective interactive diagnosis and therapy of the tumour,
**characterised by**
at least two coupling elements for coupling of diagnostic light from at least the diagnostic light source to the tumour site and therapeutic light from said therapeutic light source to said tumour site and light from said tumour site to said detector, the coupling elements in combination being
a) at least one longitudinal translatory distributor (B,C) comprising at least one translatory element (110, 111) being arranged to couple said therapeutic or diagnostic light in different constellations for different operating modes of said system by longitudinal translatory movement of said longitudinal translatory element between pre-determined positions, wherein said first optical fibres and second optical fibres are attached to said translatory element, and
at least one non-mechanical operation mode selector (310) means for optically directing either said therapeutic light or said diagnostic light to said site through said at least one first optical fibre; or
b) at least one rotary distributor (1) comprising two rotary elements (3,4) being arranged to couple light in different constellations for different operating modes of said system by rotational movement of said rotary element between pre-determined positions, wherein optical fibres are attached to said rotary elements, and
at least one non-mechanical operation mode selector (310) means for optically directing either said therapeutic light or said diagnostic light to said site through said at least one first optical fibre;
wherein the longitudinal translatory element or the rotary distributor is configured for aligning the optical fibres for transmitting or receiving said light to or from optical fibres of an opposing corresponding coupling element.

2. The system according to claim 1, **characterised by** said rotary distributor being two discs (3,4), one of which is the opposing coupling element, arranged in close proximity to each other, wherein said discs are turnable relatively to each other around a common axis (2),
each disc having holes (513) arranged on a circular line, wherein the circle radius on one disc equals the circle radius on the other disc and where the holes in one disc are equally distributed on the circle line with an angular separation of v1=(360/n1) degrees, n1 being the number of holes, and the holes in the other disc are equally distributed on the circle line with an angular separation of v2=(360/n2) degrees, wherein n2 = m x n1, and wherein m is a multiple, which yields n2 as an integer >1, and
wherein the first ends of the first optical fibres are fixed in the holes of the first disc and first ends of the second optical fibres are fixed in the holes of the second disc, whereby the first and the second optical fibres by rotation of the turnable disc are connectable to each other in said different constellations for said aligning of the optical fibres for transmitting or receiving said light to or from optical fibres of an opposing corresponding coupling element.

3. The system according to claim 1, **characterised by** said translatory distributor being a translatory element having a plurality of first optical fibres arranged for conducting light to and from the tumour site,
said opposing coupling element being a second translatory element,
the plurality of second optical fibres arranged for delivering radiation from at least one light source and/or conduction of light to at least one light sensor (12, 430), and
wherein said distributor is a distributor for distribution of light from at least one light source to the tumour site and/or from the site to at least one light sensor, wherein the distributor comprises at least one translatory element being arranged in such a manner that light is coupled in said different constellations by translatory movement of said element between pre-determined positions relative to the second translatory element for said aligning of the optical fibres for transmitting or receiving said light to or from optical fibres of an opposing corresponding coupling element.

4. The system according to claim 3, **characterised in that** each of said translatory elements has holes arranged for receiving said optical fibres and that corresponding holes on the two elements are equidistantly arranged on a straight line for said aligning of optical fibres.

5. The system according to claims 3 or 4, **characterised in that** first ends of the first optical fibres are fixed in the holes of a translatory displacement element and first ends of second optical fibres are fixed in the holes in the second translatory element, wherein the first and the second optical fibres are connectable to each other in different constellations through said translatory movement between pre-determined positions of the first translatory displacement element and the other translatory element relative each other.

6. The system according to claim 1, **characterised by** light mode selector means (310) being at least one non-mechanical light switch and/or at least one light combiner.

7. The system according to claims 1 to 6, **characterised by** said plurality of first optical fibre having a distal end, wherein said distal end is brought to said treatment site, wherein at least one first optical fibre is in use employed as a transmitter and/or a receiver for conduction of light from said at least one light source to and/or from the site for diagnosis and/or therapy.

8. The system according to claims 1 or 6, **characterised by** said diagnostic light sources being coupled to one of said mode selector means for transmission to said site, and the remaining mode selector means transmitting said diagnostic light to said at least one light detector, wherein said therapeutic light sources are inactivated.

9. The system according to claim 8, **characterised by** said diagnostic light sources being coupled to said mode selector means by means of a nxN translatory light distributor, wherein n is the number of diagnostic light sources and N is the number of mode selector means.

10. The system according to claim 9, **characterised by** said diagnostic light sources being coupled to said mode selector means by means of a translatory light distributor having n light inputs and one light output, wherein n is the number of diagnostic light sources and N is the number of mode selector means, and
a light distributor coupling said light output to one of said mode selector means.

11. The system according to claims 9 or 10, **characterised by** said mode selector means being a light combiner.

12. The system according to claims 3 to 5, **characterised by** a first longitudinally translatory light distributor element (B) coupling said optical fibres to/from said site to
a first optical fibre (7a', 160) connected to said diagnostic light sources, wherein said diagnostic light sources are connected to said first optical fibre via an light element, and to
a second longitudinally translatory light distributor element (C) coupling 2x(n-1) optical fibres to (n-1) optical fibres connected to at least one light detector, wherein n is the number of optical fibres coupled to said site, and to
N optical fibres (102) coupled to said therapeutic light sources.

13. The system according to claim 12, **characterised by** a diagnostic operation mode in which one diagnostic light source is coupled to said n first optical fibres to said site and said (n-1) first optical fibres are coupled to said light detectors by longitudinally translatory positioning said first and second translatory light distributor elements, and by
a therapeutic operation mode in which the N therapeutic light sources are coupled to corresponding N first optical fibres.

14. The system according to claim 13, **characterised in that** said light element coupling said diagnostic light sources is a light combiner.

15. The system according to any of claims 1, 9 and 12 to 14, **characterised in that** said translatory displacement element is an optical sledge.

16. The system according to claim 2, **characterised by** n1 being the number of holes in the first disc of the distributor, n1 = 6 and m = 2, yielding n2 = 12 holes in the second disc of the distributor.

17. The system according to claims 1, 2 or 16, **characterised by** a rotary light distributor element coupling said optical fibres to/from said site to
a first optical fibre (7a') connected to said diagnostic light sources, wherein said diagnostic light sources are connected to said first optical fibre via a non-mechanical light distributor element, and to
said at least one light detector from the remaining first optical fibres (7a).

18. The system according to any of the preceding claims, **characterised in that** the first optical fibres distal ends are treated by a material with temperature sensitive fluorescence emission.

19. The system according to any of the preceding claims, **characterised in that** said therapeutic light sources are light sources for coherent light of a single fixed wavelength and/or light emitting diodes.

20. The system according to claims 18-19, **characterised in that** said system is adapted to record fluorescence from said site through the same optical fibre as the one transmitting light to the site.

21. The system according to claim 20, **characterised in that** for interactive photodynamic therapy one or several of the optical fibres which are treated with the material with a temperature sensitive fluorescence emission are configured to measure the temperature at the site,
that the light which is sent to the site is adapted to heat the treatment site, and
that the intensity of the light is controllable by the measured temperature in order to regulate the temperature of the site at the individual optical fibres.

22. The system according to any of the preceding claims, **characterised by** the operation modes: interactive interstitial photo-dynamic tumour therapy, photothermal tumour therapy using hyperthermia, and tumour diagnostics, whereby these operation modes in use are alternated during the same occasion of treatment of said tumour site.

## Patentansprüche

1. System zur interaktiven interstitiellen photodynamischen oder photothermischen Tumortherapie oder Tumordiagnose beim Menschen, umfassend:
mindestens eine diagnostische (9a, 144 - 146) und eine therapeutische (101) Lichtquelle zur Emission von Licht innerhalb eines Wellenlängenbereiches von Infrarot- (IR), sichtbarem oder ultraviolettem Licht;
mindestens einen Lichtdetektor (12, 430) zur Erkennung von Licht;
und
eine Vielzahl von ersten Lichtleitfasern (6), welche so angepasst sind, dass sie Licht von oder zu einer sich in einem menschlichen Körper befindenden Tumorstelle (200) leiten, wobei eine therapeutische Lichtquelle zum Zwecke der Übertragung des therapeutischen Lichtes zur Tumorstelle über jede der Lichtleitfasern mit jeder der Lichtleitfasern verbunden ist, wobei die diagnostischen Lichtquellen (9a) während dieses Vorganges inaktiviert werden, und wobei die distalen Enden der Lichtleitfasern an unterschiedlichen interstitiellen Positionen der Tumorstelle positioniert werden, um eine effektive interaktive Diagnose und Therapie des Tumors zu ermöglichen,
**gekennzeichnet durch**
mindestens zwei Kopplungselemente zur Kopplung von mindestens diagnostischem Licht von der diagnostischen Lichtquelle an die Tumorstelle und des therapeutischen Lichts von der therapeutischen Lichtquelle an die Tumorstelle, sowie von Licht von der Tumorstelle an den Detektor, wobei die Kopplungselemente in Kombination bestehen aus
a) mindestens einem in Längsrichtung translatorischen Verteiler (B, C), welcher mindestens ein Translationselement (110, 111) umfasst, das so angeordnet ist, dass es das therapeutische und diagnostische Licht für verschiedene Betriebsmodi des Systems **durch** eine in Längsrichtung translatorische Bewegung des in Längsrichtung translatorischen Elements zwischen festgelegten Positionen in verschiedenen Konstellationen koppelt,
wobei die ersten Lichtleitfasern und die zweiten Lichtleitfasern mit dem translatorischen Element verbunden sind, und
mindestens einem nicht-mechanischen Betriebswahlmittel (310) zur optischen Leitung des therapeutischen Lichts oder des diagnostischen Lichts zur Behandlungsstelle **durch** die mindestens eine erste Lichtleitfaser; oder
b) mindestens einem Rundverteiler (1), welcher zwei Drehelemente (3, 4) umfasst, die so angeordnet sind, dass sie **durch** eine Drehbewegung des Drehelements zwischen festgelegten Positionen Licht für verschiedene Betriebsmodi des Systems in verschiedenen Konstellationen koppeln, wobei Lichtleitfasern an den Drehelementen angebracht sind, und
mindestens einem nicht-mechanischen Betriebswahlmittel (310) zur optischen Leitung des therapeutischen Lichts oder des diagnostischen Lichts **durch** die mindestens eine erste Lichtleitfaser zur Behandlungsstelle;
wobei das Längstranslationselement oder der Rundverteiler zum Zwecke der Anpassung der Lichtleitfasern für die Übertragung oder den Empfang des Lichtes zu oder von den Lichtleitfasern eines gegenüberliegenden dazugehörigen Kupplungselements konfiguriert ist.

2. System gemäß Anspruch 1, **dadurch gekennzeichnet, dass** es sich bei dem Rundverteiler um zwei Scheiben (3, 4) handelt, die nah aneinander angeordnet sind und von denen eine das gegenüberliegende Kupplungselement darstellt, wobei die Scheiben in Relation zueinander um eine gemeinsame Achse (2) herum drehbar sind,
wobei jede Scheibe Löcher (513) aufweist, welche in einer runden Linie angeordnet sind, wobei der Kreisradius auf einer Scheibe dem Kreisradius auf der anderen Scheibe entspricht, und wobei die Löcher in einer Scheibe mit einem Winkelabstand von v1=(360/n1) Grad gleichmäßig auf der Kreislinie verteilt sind, wobei n1 die Anzahl der Löcher darstellt, und die Löcher in der anderen Scheibe mit einem Winkelabstand von v2 = (360/n2) Grad gleichmäßig auf der Kreislinie verteilt sind, wobei n2 = m x n1 gilt, und wobei es sich bei m um ein Vielfaches handelt, so dass sich n2 als eine Ganzzahl > 1 ergibt, und
wobei die ersten Enden der ersten Lichtleitfasern in den Löchern der ersten Scheibe befestigt sind und die ersten Enden der zweiten Lichtleitfasern in den Löchern der zweiten Scheibe befestigt sind, wobei sich die ersten und die zweiten Lichtleitfasern durch Drehung der drehbaren Scheibe zum Zwecke der Anpassung der Lichtleitfasern für die Übertragung oder den Empfang des Lichtes zu oder von den Lichtleitfasern eines gegenüberliegenden dazugehörigen Kupplungselements in den verschiedenen Konstellationen miteinander verbinden lassen.

3. System gemäß Anspruch 1, **dadurch gekennzeichnet, dass** es sich bei dem translatorischen Verteiler um ein Translationselement handelt, welches eine Vielzahl von ersten Lichtleitfasern aufweist, die so angeordnet sind, dass sie Licht von oder zu der Tumorstelle leiten,
wobei es sich bei dem gegenüberliegenden Kupplungselement um ein zweites Translationselement handelt,
wobei die Vielzahl von zweiten Lichtleitfasern so angeordnet ist, dass sie die Zuführung von Strahlung, die von mindestens einer Lichtquelle stammt, und/oder die Leitung von Licht zu mindestens einem Lichtsensor (12, 430) hin ermöglicht, und
wobei es sich bei dem Verteiler um einen Verteiler zur Verteilung mindestens desjenigen Lichts handelt, welches von einer Lichtquelle an die Tumorstelle und/oder von der Behandlungsstelle an mindestens einen Lichtsensor geht, wobei der Verteiler mindestens ein Translationselement umfasst, welches so angeordnet ist, dass Licht in den verschiedenen Konstellationen durch die Translationsbewegung des Elements zwischen festgelegten Positionen in Relation zu einem zweiten Translationselement gekoppelt wird, was zum Zwecke der Anpassung der Lichtleitfasern für die Übertragung oder den Empfang von Licht zu oder von den Lichtleitfasern eines gegenüberliegenden dazugehörigen Kupplungselements erfolgt.

4. System gemäß Anspruch 3, **dadurch gekennzeichnet, dass** jedes der Translationselemente Löcher aufweist, die der Aufnahme der Lichtleitfasern dienen, und dass die einander entsprechenden Löcher auf den zwei Elementen zur Anpassung der Lichtleitfasern in gleichen Abständen auf einer geraden Linie angeordnet ist.

5. System gemäß einem der Ansprüche 3 oder 4, **dadurch gekennzeichnet, dass** die ersten Enden der ersten Lichtleitfasern in den Löchern eines translatorischen Verdrängungselements befestigt sind und die ersten Enden der zweiten Lichtleitfasern in den Löchern im zweiten Translationselement befestigt sind, wobei die ersten und zweiten Lichtleitfasern sich durch die Translationsbewegung zwischen festgelegten Positionen des ersten translatorischen Verdrängungselements und des anderen translatorischen Elements in Relation zueinander in verschiedenen Konstellationen miteinander verbinden lassen.

6. System gemäß Anspruch 1, **dadurch gekennzeichnet, dass** das Mittel zur Lichtbetriebsartenwahl (310) aus mindestens einem nicht-mechanischen Lichtschalter und/oder mindestens einem Lichtkombinator besteht.

7. System gemäß einem der Ansprüche 1 bis 6, **dadurch gekennzeichnet, dass** die Vielzahl von ersten Lichtleitfasern ein distales Ende aufweist, wobei das distale Ende an die Behandlungsstelle gebracht wird, und wobei mindestens eine erste Lichtleitfaser beim Betrieb als ein Sender und/oder ein Empfänger zur Leitung von Licht von der mindestens einen Lichtquelle zu und/oder von der Behandlungsstelle verwendet wird, was der Diagnose und/oder Therapie dient.

8. System gemäß Anspruch 1 oder 6, **dadurch gekennzeichnet, dass** die diagnostischen Lichtquellen zum Zwecke der Übertragung an die Behandlungsstelle an eines der Betriebswahlmittel gekoppelt sind, und dass die verbleibenden Betriebswahlmittel das diagnostische Licht zu dem mindestens einen Lichtdetektor hin übertragen, wobei die therapeutischen Lichtquellen inaktiviert sind.

9. System gemäß Anspruch 8, **dadurch gekennzeichnet, dass** die diagnostischen Lichtquellen mittels eines translatorischen nxN-Lichtverteilers an die Betriebswahlmittel gekoppelt sind, wobei n die Anzahl der diagnostischen Lichtquellen und N die Anzahl der Betriebswahlmittel darstellt.

10. System gemäß Anspruch 9, **dadurch gekennzeichnet, dass** die diagnostischen Lichtquellen mittels eines translatorischen Lichtverteilers, welcher n Lichteingänge und einen Lichtausgang hat, an die Betriebswahlmittel gekoppelt sind, wobei n die Anzahl der diagnostischen Lichtquellen und N die Anzahl der Betriebswahlmittel darstellt, und dass
ein Lichtverteiler den Lichtausgang an eines der Betriebswahlmittel koppelt.

11. System gemäß einem der Ansprüche 9 oder 10, **dadurch gekennzeichnet, dass** es sich bei dem Betriebswahlmittel um einen Lichtkombinator handelt.

12. System gemäß einem der Ansprüche 3 bis 5, **dadurch gekennzeichnet, dass** ein erstes in Längsrichtung translatorisches Lichtverteilerelement (B) die zu/von der Behandlungsstelle verlaufenden Lichtleitfasern koppelt an
eine erste Lichtleitfaser (7a', 160), welche mit den diagnostischen Lichtquellen verbunden ist, wobei die Verbindung der diagnostischen Lichtquellen mit der ersten Lichtleitfaser über ein Lichtelement hergestellt wird, und an
ein zweites in Längsrichtung translatorisches Lichtverteilerelement (C), welches 2x (n-1) Lichtleitfasern an (n-1) Lichtleitfasern koppelt, die mit mindestes einem Lichtdetektor verbunden sind, wobei n die Anzahl der Lichtleitfasern ist, welche an die Behandlungsstelle gekoppelt sind, und an
N Lichtleitfasern (102), welche an die therapeutischen Lichtquellen gekoppelt sind.

13. System gemäß Anspruch 12, **gekennzeichnet durch** einen diagnostischen Betriebsmodus, bei dem eine diagnostische Lichtquelle an n erste Lichtleitfasern an die Behandlungsstelle gekoppelt ist, und bei dem die (n-1) ersten Lichtleitfasern **durch** translatorische längsgerichtete Positionierung der ersten und zweiten translatorischen Lichtverteilerelemente an die Lichtdetektoren gekoppelt sind, sowie **durch** einen therapeutischen Betriebsmodus, in welchem die N therapeutischen Lichtquellen an die N dazugehörigen ersten Lichtleitfasern gekoppelt sind.

14. System gemäß Anspruch 13, **dadurch gekennzeichnet, dass** es sich bei dem Lichtelement, durch welches die Kopplung der diagnostischen Lichtquellen erfolgt, um einen Lichtkombinator handelt.

15. System gemäß einem beliebigen der Ansprüche 1, 9 und 12 bis 14, **dadurch gekennzeichnet, dass** es sich bei dem translatorischen Verdrängungselement um einen optischen Schlitten handelt.

16. System gemäß Anspruch 2, **dadurch gekennzeichnet, dass** n1 die Anzahl der Löcher in der ersten Scheibe des Verteilers bezeichnet, wobei n1 = 6 und m = 2 gilt, wodurch sich für die zweite Scheibe des Verteilers n2 = 12 Löcher ergeben.

17. System gemäß einem der Ansprüche 1, 2 oder 16, **dadurch gekennzeichnet, dass** ein drehbares Lichtverteilerelement die zu/von der Behandlungsstelle verlaufenden Lichtleitfasern koppelt an
eine erste Lichtleitfaser (7a'), welche verbunden ist mit den diagnostischen Lichtquellen, wobei die Verbindung zwischen den diagnostischen Lichtquellen und der ersten Lichtleitfaser über ein nicht-mechanisches Lichtverteilerelement hergestellt wird, und an
mindestens einen Lichtdetektor von den verbleibenden ersten Lichtleitfasern (7a).

18. System gemäß einem beliebigen der vorangegangenen Ansprüche, **dadurch gekennzeichnet, dass** die distalen Enden der ersten Lichtleitfasern behandelt sind mit einem Material, das sich durch temperaturempfindliche Fluoreszenzemission auszeichnet.

19. System gemäß einem beliebigen der vorangegangenen Ansprüche, **dadurch gekennzeichnet, dass** es sich bei den therapeutischen Lichtquellen um Lichtquellen für kohärentes Licht einer einzelnen festgesetzten Wellenlänge und/oder um lichtemittierende Dioden handelt.

20. System gemäß einem der Ansprüche 18 bis 19, **dadurch gekennzeichnet, dass** das System so angepasst ist, dass von der Behandlungsstelle kommende Fluoreszenz durch dieselbe Lichtleitfaser aufgezeichnet wird, durch die auch die Übertragung des Lichtes zur Behandlungsstelle erfolgt.

21. System gemäß Anspruch 20, **dadurch gekennzeichnet, dass** für die interaktive photodynamische Therapie eine oder mehrere der Lichtleitfasern, welche mit dem temperaturabhängig fuoreszenzemittierenden Material behandelt worden sind, so konfiguriert sind, dass sie die Temperatur an der Behandlungsstelle messen,
dass das Licht, welches an die Behandlungsstelle gesendet wird, so beschaffen ist, dass es die Behandlungsstelle erwärmt, und
dass sich die Intensität des Lichts anhand der gemessenen Temperatur kontrollieren lässt, so dass die Temperatur der Behandlungsstelle an den einzelnen Lichtleitfasern reguliert werden kann.

22. System gemäß einem beliebigen der vorangegangenen Ansprüche, **gekennzeichnet durch** die Betriebsmodi:
interaktive interstitielle photodynamische Tumortherapie, photothermische Tumortherapie unter Verwendung von Hyperthermie, und Tumordiagnostik, wobei diese verwendeten Betriebsmodi während derselben Behandlungssitzung der Tumorstelle im Wechsel angewendet werden können.

## Revendications

1. Système destiné à une thérapie de tumeur photothermique ou photodynamique interstitielle interactive ou un diagnostic de tumeur d'un être humain, comprenant
au moins une source de lumière de diagnostic (9a, 144-146) et une source de lumière thérapeutique (101) destinées à une émission de lumière au sein de la plage de longueurs d'onde de la lumière infrarouge (IR), visible ou ultraviolette ;
au moins un détecteur de lumière (12, 430) destiné à une détection de lumière ; et
une pluralité de premières fibres optiques (6) conçues pour acheminer de la lumière à destination ou provenant d'un site tumoral (200) dudit être humain, grâce à quoi une source de lumière thérapeutique est couplée à chacune desdites fibres optiques en vue d'une transmission de ladite lumière thérapeutique jusqu'audit site tumoral par l'intermédiaire de chacune desdites fibres optiques, au cours de laquelle les sources de lumière de diagnostic (9a) sont désactivées, grâce à quoi des extrémités distales des fibres optiques sont positionnées à différentes positions interstitielles du site tumoral de manière à permettre un diagnostic et une thérapie interactifs efficaces de la tumeur,
**caractérisé par**
au moins deux éléments de couplage destinés à un couplage de lumière de diagnostic provenant de la au moins une source de lumière de diagnostic à destination du site tumoral et de lumière thérapeutique provenant de ladite source de lumière thérapeutique à destination dudit site tumoral et de lumière provenant dudit site tumoral à destination dudit détecteur, les éléments de couplage en combinaison étant
a) au moins un répartiteur par translation longitudinale (B, C) comprenant au moins un élément de translation (110, 111) qui est agencé de manière à coupler ladite lumière thérapeutique ou de diagnostic selon différentes constellations pour différents modes de fonctionnement dudit système par un mouvement de translation longitudinale dudit élément de translation longitudinale entre des positions prédéterminées, où lesdites premières fibres optiques et deuxièmes fibres optiques sont fixées audit élément de translation, et
au moins un moyen de sélecteur de mode de fonctionnement non mécanique (310) destiné à orienter de manière optique soit ladite lumière thérapeutique, soit ladite lumière de diagnostic vers ledit site par l'intermédiaire de ladite au moins une première fibre optique ; ou
b) au moins un répartiteur rotatif (1) comprenant deux éléments rotatifs (3, 4) qui sont agencés de manière à coupler de la lumière selon différentes constellations pour différents modes de fonctionnement dudit système par un mouvement rotatif dudit élément rotatif entre des positions prédéterminées, où des fibres optiques sont fixées auxdits éléments rotatifs, et
au moins un moyen de sélecteur de mode de fonctionnement non mécanique (310) destiné à orienter de manière optique soit ladite lumière thérapeutique, soit ladite lumière de diagnostic vers ledit site par l'intermédiaire de ladite au moins une première fibre optique ;
où l'élément de translation longitudinale ou le répartiteur rotatif est configuré pour aligner les fibres optiques en vue d'une transmission ou d'une réception de ladite lumière à destination ou provenant des fibres optiques d'un élément de couplage correspondant opposé.

2. Système selon la revendication 1, **caractérisé par** ledit répartiteur rotatif qui correspond à deux disques (3, 4), l'un d'entre eux étant l'élément de couplage opposé, agencés à proximité étroite l'un par rapport à l'autre, où lesdits disques peuvent être tournés relativement l'un par rapport à l'autre autour d'un axe commun (2),
chaque disque comportant des trous (513) agencés sur une ligne circulaire, où le rayon de cercle d'un premier disque est égal au rayon de cercle de l'autre disque et où les trous dans un premier disque sont répartis de manière égale sur la ligne de cercle avec une séparation angulaire de v1 = (360 / n1) degrés, n1 étant le nombre de trous, et les trous dans l'autre disque sont répartis de manière égale sur la ligne de cercle avec une séparation angulaire de v2 = (360 / n2) degrés, où n2 = m x n1, et où m est un multiple, qui donne n2 comme un entier > 1, et
où les premières extrémités des premières fibres optiques sont fixées dans les trous du premier disque et les premières extrémités des deuxièmes fibres optiques sont fixées dans les trous du deuxième disque, grâce à quoi les premières et deuxièmes fibres optiques par rotation du disque pouvant être tourné peuvent être connectées les unes aux autres selon lesdites différentes constellations pour ledit alignement des fibres optiques en vue d'une transmission ou d'une réception de ladite lumière à destination ou provenant des fibres optiques d'un élément de couplage correspondant opposé.

3. Système selon revendication 1, **caractérisé par** ledit répartiteur par translation qui est un élément de translation présentant une pluralité de premières fibres optiques agencées de manière à acheminer de la lumière à destination et provenant du site tumoral,
ledit élément de couplage opposé étant un deuxième élément de translation,
la pluralité de deuxièmes fibres optiques agencées de manière à délivrer un rayonnement provenant d'au moins une source de lumière et/ou acheminer de la lumière à destination d'au moins un capteur de lumière (12, 430), et
où ledit répartiteur est un répartiteur destiné à répartir de la lumière provenant d'au moins une source de lumière à destination du site tumoral et/ou provenant du site à destination d'au moins un capteur de lumière, où le répartiteur comprend au moins un élément de translation qui est agencé de telle manière que de la lumière est couplée selon lesdites différentes constellations par un mouvement de translation dudit élément entre des positions prédéterminées par rapport au deuxième élément de translation pour ledit alignement des fibres optiques en vue d'une transmission ou d'une réception de ladite lumière à destination ou provenant des fibres optiques d'un élément de couplage correspondant opposé.

4. Système selon la revendication 3, **caractérisé en ce que** chacun desdits éléments de translation comporte des trous agencés de manière à recevoir lesdites fibres optiques et **en ce que** les trous correspondants sur les deux éléments sont agencés de manière équidistante sur une ligne droite pour ledit alignement de fibres optiques.

5. Système selon les revendications 3 ou 4, **caractérisé en ce que** les premières extrémités des premières fibres optiques sont fixées dans les trous d'un élément de déplacement par translation et les premières extrémités des deuxièmes fibres optiques sont fixées dans les trous dans le deuxième élément de translation, où les premières et les deuxièmes fibres optiques peuvent être connectées les unes aux autres selon différentes constellations par l'intermédiaire dudit mouvement de translation entre des positions prédéterminées du premier élément de déplacement par translation et de l'autre élément de translation l'un par rapport à l'autre.

6. Système selon la revendication 1, **caractérisé par** un moyen de sélecteur de mode de lumière (310) qui est au moins un commutateur de lumière non mécanique et/ou au moins un élément de combinaison de lumière.

7. Système selon les revendications 1 à 6, **caractérisé par** ladite pluralité de premières fibres optiques présentant une extrémité distale, où ladite extrémité distale est amenée jusqu'audit site de traitement, où au moins une première fibre optique est en cours d'utilisation employée en tant que transmetteur et/ou récepteur en vue d'un acheminement de lumière provenant de ladite au moins une source de lumière à destination de et/ou provenant du site pour un diagnostic et/ou une thérapie.

8. Système selon les revendications 1 ou 6, **caractérisé par** lesdites sources de lumière de diagnostic qui sont couplées à l'un desdits moyens de sélecteur de mode en vue d'une transmission audit site, et le moyen de sélecteur de mode restant transmettant ladite lumière de diagnostic audit au moins un détecteur de lumière, où lesdites sources de lumière thérapeutique sont désactivées.

9. Système selon la revendication 8, **caractérisé par** lesdites sources de lumière de diagnostic qui sont couplées auxdits moyens de sélecteur de mode au moyen d'un répartiteur de lumière par translation n x N, où n est le nombre de sources de lumière de diagnostic et N est le nombre de moyens de sélecteur de mode.

10. Système selon la revendication 9, **caractérisé par** lesdites sources de lumière de diagnostic qui sont couplées auxdits moyens de sélecteur de mode au moyen d'un répartiteur de lumière par translation comportant n entrées de lumière et une seule sortie de lumière, où n est le nombre de sources de lumière de diagnostic et N est le nombre de moyens de sélecteur de mode, et
un répartiteur de lumière couplant ladite sortie de lumière à un desdits moyens de sélecteur de mode.

11. Système selon les revendications 9 ou 10, **caractérisé par** lesdits moyens de sélecteur de mode qui sont un élément de combinaison de lumière.

12. Système selon les revendications 3 à 5, **caractérisé par** un premier élément de répartiteur de lumière par translation de manière longitudinale (B) couplant lesdites fibres optiques à destination/provenant dudit site à
une première fibre optique (7a', 160) connectée auxdites sources de lumière de diagnostic, où lesdites sources de lumière de diagnostic sont connectées à ladite première fibre optique via un élément de lumière, et à
un deuxième élément de répartiteur de lumière par translation de manière longitudinale (C) couplant 2 x (n - 1) fibres optiques à (n - 1) fibres optiques connectées à au moins un détecteur de lumière, où n est le nombre de fibres optiques couplées audit site, et à
N fibres optiques (102) couplées auxdites sources de lumière thérapeutique.

13. Système selon la revendication 12, **caractérisé par** un mode de fonctionnement de diagnostic dans lequel une source de lumière de diagnostic est couplée auxdites n premières fibres optiques vers ledit site et lesdites (n - 1) premières fibres optiques sont couplées auxdits détecteurs de lumière par un positionnement par translation de manière longitudinale desdits premier et deuxième éléments de répartiteur de lumière par translation, et par
un mode de fonctionnement thérapeutique dans lequel les N sources de lumière thérapeutique sont couplées à N premières fibres optiques correspondantes.

14. Système selon la revendication 13, **caractérisé en ce que** ledit élément de lumière couplant lesdites sources de lumière de diagnostic est un élément de combinaison de lumière.

15. Système selon l'une quelconque des revendications 1, 9 et 12 à 14, **caractérisé en ce que** ledit élément de déplacement par translation est un traîneau optique.

16. Système selon la revendication 2, **caractérisé par** n1 qui est le nombre de trous dans le premier disque du répartiteur, n1 = 6 et m = 2, ce qui donne n2 = 12 trous dans le deuxième disque du répartiteur.

17. Système selon les revendications 1, 2 ou 16, **caractérisé par** un élément de répartiteur de lumière rotatif couplant lesdites fibres optiques à destination/provenant dudit site à
une première fibre optique (7a') connectée auxdites sources de lumière de diagnostic, où lesdites sources de lumière de diagnostic sont connectées à ladite première fibre optique via un élément de répartiteur de lumière non mécanique, et à
ledit au moins un détecteur de lumière à partir des premières fibres optiques restantes (7a).

18. Système selon l'une quelconque des revendications précédentes, **caractérisé en ce que** les extrémités distales des premières fibres optiques sont traitées par un matériau présentant une émission de fluorescence sensible à la température.

19. Système selon l'une quelconque des revendications précédentes, **caractérisé en ce que** lesdites sources de lumière thérapeutique sont des sources de lumière pour une lumière cohérente d'une seule longueur d'onde fixée et/ou des diodes électroluminescentes.

20. Système selon les revendications 18 à 19, **caractérisé en ce que** ledit système est conçu pour enregistrer la fluorescence provenant dudit site par l'intermédiaire de la même fibre optique que celle transmettant de la lumière jusqu'au site.

21. Système selon la revendication 20, **caractérisé en ce que** pour une thérapie photodynamique interactive, une ou plusieurs des fibres optiques qui sont traitées avec le matériau présentant une émission de fluorescence sensible à la température sont configurées pour mesurer la température au niveau du site,
**en ce que** la lumière qui est envoyée au site est adaptée pour chauffer le site de traitement, et
**en ce que** l'intensité de la lumière peut être contrôlée par la température mesurée de manière à réguler la température du site au niveau des fibres optiques individuelles.

22. Système selon l'une quelconque des revendications précédentes, **caractérisé par** les modes de fonctionnement :
thérapie de tumeur photodynamique interstitielle interactive, thérapie de tumeur photothermique utilisant une hyperthermie, et diagnostics de tumeur, grâce à quoi ces modes de fonctionnement en cours d'utilisation sont alternés au cours de la même occasion de traitement dudit site tumoral.
